# EUROPEAN PATENT APPLICATION

(11) **EP 4 737 472 A1**
(43) Date of publication of application: **06.05.2026**
(21) Application number: 24832084.8
(22) Date of filing: 28.06.2024
(51) Int. Cl.: C07K 1/14, C12N 9/88, C12N 15/09, C12P 21/00, C12P 21/02

(54) **METHOD FOR REMOVING PLANT-DERIVED SOLUBLE PROTEIN FROM PLANT-DERIVED EXTRACT**

(30) Priority: 29.06.2023 JP 2023106995; 27.11.2023 JP 2023200214
(71) Applicant: National Institute of Advanced Industrial Science and Technology, Chiyoda-ku Tokyo 100-8921 (JP); Chiyoda Corporation, Kanagawa 220-8765 (JP)
(72) Inventor: MATSUO, Kouki, Tsukuba-shi, Ibaraki 305-8560 (JP); MATSUSHIMA, Akito, Yokohama-shi, Kanagawa 220-8765 (JP); NISHIDA, Naoko, Yokohama-shi, Kanagawa 220-8765 (JP)
(74) Representative: Gulde & Partner
(86) International application number: PCT/JP2024/023476
(87) International publication number: WO 2025/005226

(57) **Abstract**

The present invention provides a method for removing a plant-derived soluble protein from a plant-derived extract, the method comprising the following steps: (1) a step for insolubilizing the plant-derived soluble protein by freezing and thawing the plant-derived extract, and (2) a step for removing the insolubilized protein from the extract.

## Description

### Technical Field

The present invention relates to a method for removing a plant-derived soluble protein from a plant-derived extract, and the like.

### Background Art

Technology for producing substances using organisms such as plants, animals, or microorganisms is capable of material production from biomass, which does not depend on fossil resources. Therefore, it is anticipated to lead to the realization of a carbon-circulating society and manufacturing innovation toward sustainable economic growth. Among these, substance production by plants using genetic recombination technology has the potential to express proteins that are difficult to produce due to complex structures or high molecular weights, and is seen as promising for utilization in fields such as pharmaceuticals, food, and healthcare. However, there is still little commercial track record, and technologies for mass production have not been established. In particular, the separation and purification of the target expressed protein requires multiple reagents and multi-step operations (Patent Literature 1), and there is a great demand for an efficient purification method. In particular, there is a great demand for a purification method that can efficiently remove a plant-derived soluble protein, such as RuBisCO, which are contained in large quantities in plant-derived extracts.

### Citation List

### Patent Literature

Patent Literature 1: JP 6560495 B

### Summary of Invention

### Technical Problem

An object of the present invention is to provide a method for removing a plant-derived soluble protein, which is present in large quantities, from a plant-derived extract.

### Solution to Problem

The present inventors centrifuged crude extracts of wild-type *Nicotiana benthamiana* and *N. benthamiana* into which a green fluorescent protein (GFP) gene had been introduced, separating them into a supernatant (S1) and a precipitate (P1). Furthermore, S1 was subjected to freeze-thaw treatment, centrifuged, and separated into a supernatant (S2) and a precipitate (P2). Subsequently, the soluble proteins contained in S1 and S2 were separated by SDS-PAGE, and the polyacrylamide gel was stained with Coomassie Blue. As a result, it was confirmed that Ribulose-1,5-bisphosphate carboxylase/oxygenase (RuBisCO), a plant-derived soluble protein confirmed to be present in large quantities in S1, had almost disappeared in S2. At the same time, by subjecting S1, from which the precipitate P1 had already been removed by centrifugation, to freeze-thaw treatment and centrifugation, a new precipitate P2 was formed, which was considered to be a new insoluble fraction containing RuBisCO. Furthermore, analysis of the amount of GFP contained in S1 and S2 by Western blotting revealed that the freeze-thaw treatment did not affect the solubility of GFP, which remained in the supernatant.

In addition, the inventors found that even a short freezing treatment of about 10 minutes could remove some plant-derived soluble proteins, and a 60-minute freezing treatment could remove a sufficient amount of RuBisCO; the present invention could be implemented even when using GUS protein or human FGF1 as the exogenous protein; and that many types of plant-derived soluble proteins other than RuBisCO could be removed by the freeze-thaw treatment. From the above, it became clear that the freeze-thaw treatment insolubilizes plant-derived soluble proteins, including RuBisCO, while not affecting the solubility of exogenous proteins such as GFP, GUS, or FGF1, consequently enabling the separation of both. Furthermore, the freezing time did not affect the above-mentioned effects. The present inventors conducted further research based on these findings and completed the present invention.

That is, the present invention is as follows.
[1] A method for removing a plant-derived soluble protein from a plant-derived extract, the method comprising the following steps:
   (1) a step for insolubilizing the plant-derived soluble protein by freezing and thawing the plant-derived extract, and
   (2) a step for removing the insolubilized protein from the extract.
[2] The method of [1], wherein the plant-derived soluble protein comprises RuBisCO.
[3] The method of [1] or [2], wherein the plant is a plant belonging to the genus Nicotiana.
[4] The method of [3], wherein the plant belonging to the genus *Nicotiana* is *Nicotiana benthamiana*.
[5] The method of any one of [1] to [4], wherein the plant expresses an exogenous soluble protein which is not insolubilized by freezing and thawing.
[6] The method of any one of [1] to [5], wherein a freezing period is 1 day or more.
[7] The method of any one of [1] to [6], wherein a freezing temperature is -20°C or lower.
[8] The method of any one of [1] to [7], wherein the removing of the insolubilized protein from the extract is performed by centrifugation or filtration.
[9] The method of any one of [1] to [8], further comprising a step of removing insoluble substances from the plant-derived extract before the step (1).
[10] The method of [9], wherein the removing of insoluble substances from the plant-derived extract is performed by centrifugation or filtration.

### Advantageous Effects of Invention

According to the method described in the present specification for separating plant-derived soluble proteins, including RuBisCO, from a plant-derived extract, whereas conventionally, separation and purification of exogenous soluble proteins expressed in plants required multiple reagents and multi-step operations, by applying a freeze-thaw treatment, plant-derived soluble proteins, including the dominant protein in plants, RuBisCO, can be removed by insolubilization. This can increase the proportional presence of the exogenous soluble protein upstream in the purification process. As a result, it becomes possible to shorten the purification step for the exogenous soluble protein, and the present invention can be useful in the bioindustry field.

### Brief Description of Drawings

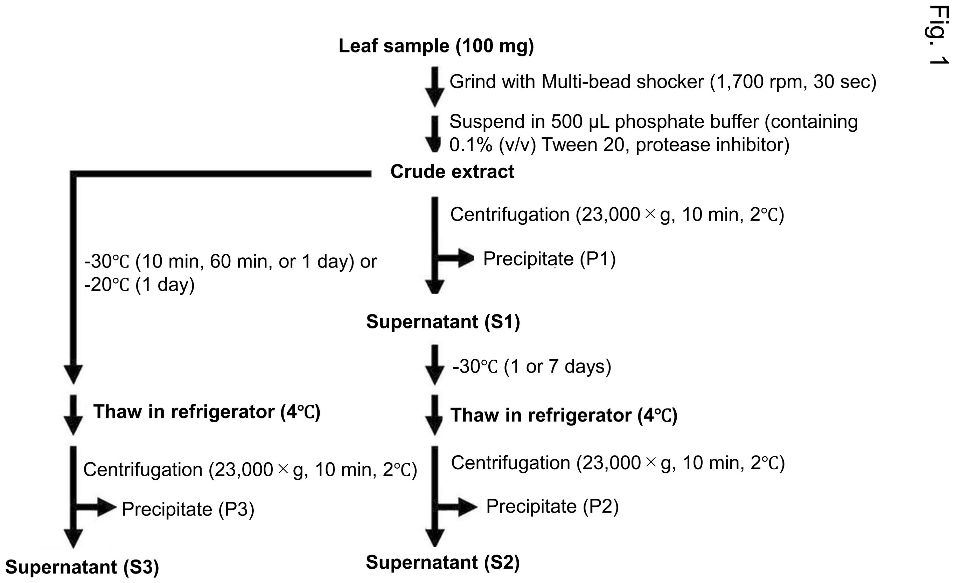

Fig. 1 shows a flowchart of the extraction treatment and freeze-thaw treatment of *N. benthamiana* leaf samples. S1 indicates the supernatant obtained by centrifuging an unfrozen crude extract; S2 indicates the supernatant obtained by centrifuging S1 after freezing for 1 day or 7 days and then thawing; and S3 indicates the supernatant obtained by centrifuging a crude extract that was frozen for 1 day and then thawed.

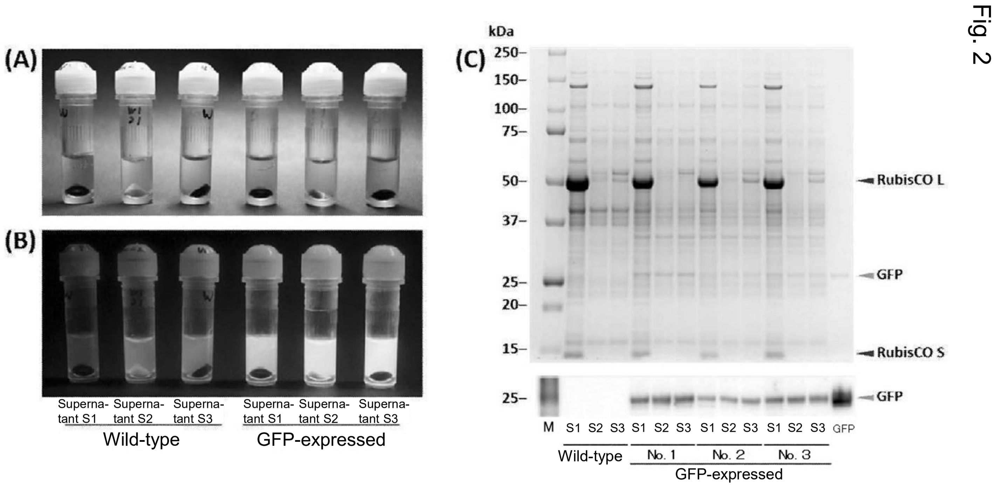

Fig. 2 shows the results of subjecting crude extracts and supernatants from wild-type *N. benthamiana* leaves and *N. benthamiana* leaves (n=3) into which the GFP gene was introduced to freeze-thaw treatment. S1 to S3 and P1 to P3 of wild-type and GFP gene-introduced types were photographed (A) under visible light and (B) under blue LED. (C) Proteins in S1 to S3 were separated by SDS-PAGE, followed by Coomassie Blue staining (top) or Western blot analysis (bottom). M indicates the molecular weight marker, RuBisCO L indicates the RuBisCO large subunit, and RuBisCO S indicates the RuBisCO small subunit.

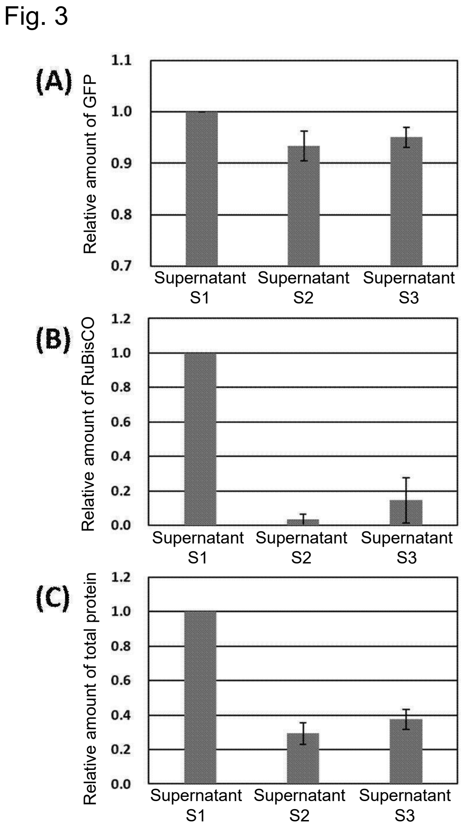

Fig. 3 shows the relative amounts of proteins contained in each supernatant. (A) shows the relative amount of recombinant GFP estimated from Western blot analysis. The p-values (significance probabilities) are 2.96×10⁻³ for S2 and 2.42×10⁻³ for S3, respectively. (B) The relative amount of RuBisCO was estimated from the band intensity of the Coomassie Blue-stained polyacrylamide gel. The p-values were 3.01×10⁻⁴ for S2 and 6.46×10⁻³ for S3, respectively. (C) The relative amount of total protein was estimated from the band intensity of the Coomassie Blue-stained polyacrylamide gel. Error bars represent standard deviation (n=3).

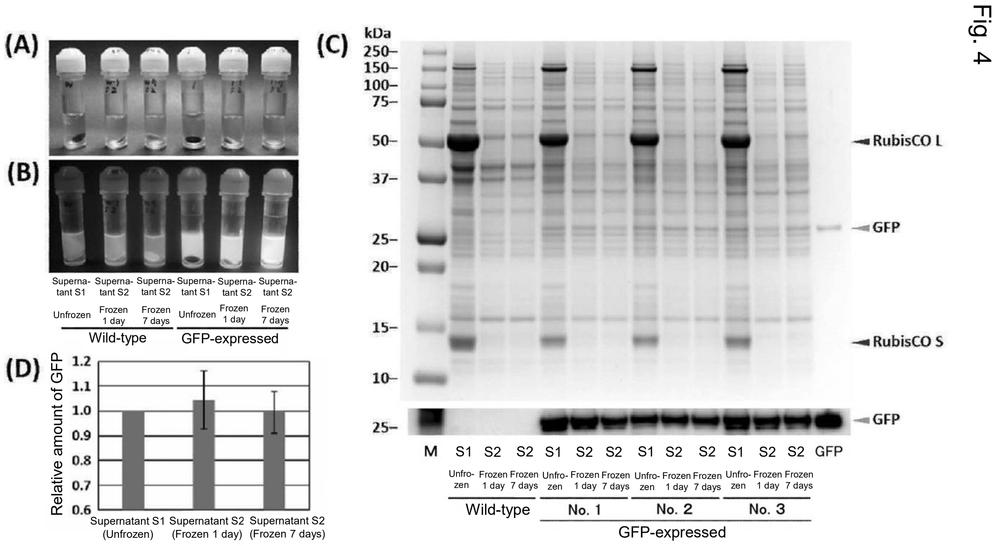

Fig. 4 shows the results of subjecting the supernatants of extracts from wild-type *N. benthamiana* leaves and GFP gene-introduced *N. benthamiana* leaves (n=3) to freeze-thaw treatment. S1 and S2 of wild-type and GFP gene-introduced types were photographed (A) under visible light and (B) under blue LED. (C) Proteins in S1 and S2 were separated by SDS-PAGE, followed by Coomassie Blue staining (top) or Western blot analysis (bottom). (D) shows the relative amounts of GFP in S1, S2 (frozen 1 day), and S2 (frozen 7 days). The p-values (significance probabilities) were 0.294 for S2 (frozen 1 day) and 0.456 for S2 (frozen 7 days), respectively, showing no significant difference. M indicates the molecular weight marker, RuBisCO L indicates the RuBisCO large subunit, and RuBisCO S indicates the RuBisCO small subunit.

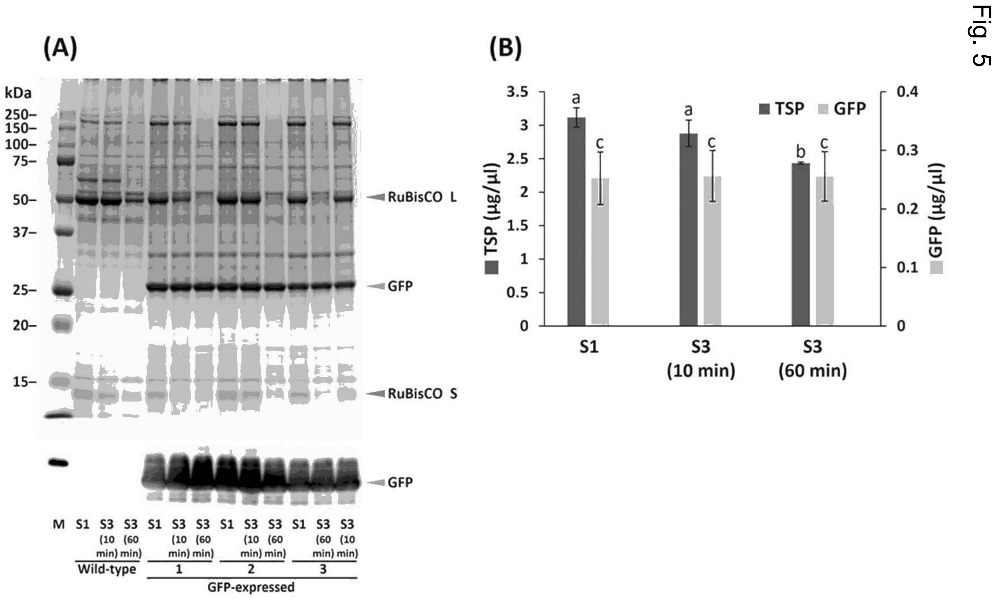

Fig. 5 is a diagram showing the effect of short-time freeze-thaw treatment. Crude extracts from wild-type and three independent GFP-expressing leaves were frozen at - 30°C for 10 minutes or 60 minutes, and then thawed. (A) Proteins were separated by SDS-PAGE and detected by Coomassie Blue staining (top) or Western blot (bottom). M is the molecular weight marker, RuBisCO L is the RuBisCO large subunit, RuBisCO S is the RuBisCO small subunit, S1 is the unfrozen supernatant, and S3 is the supernatant of the crude extract frozen at -30°C for 10 or 60 minutes. (B) Concentration of Total Soluble Proteins (often referred to as TSP) and GFP in the corresponding supernatants.

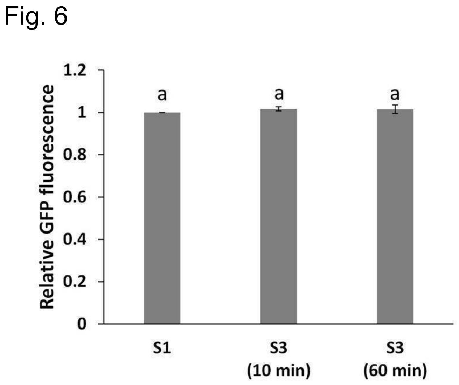

Fig. 6 is a diagram showing the effect of short-time freeze-thaw treatment on the function of GFP. It shows the relative GFP fluorescence intensity of S1 (unfrozen) and S3 (supernatant of crude juice frozen at -30°C for 10 or 60 minutes) from GFP-expressing plants.

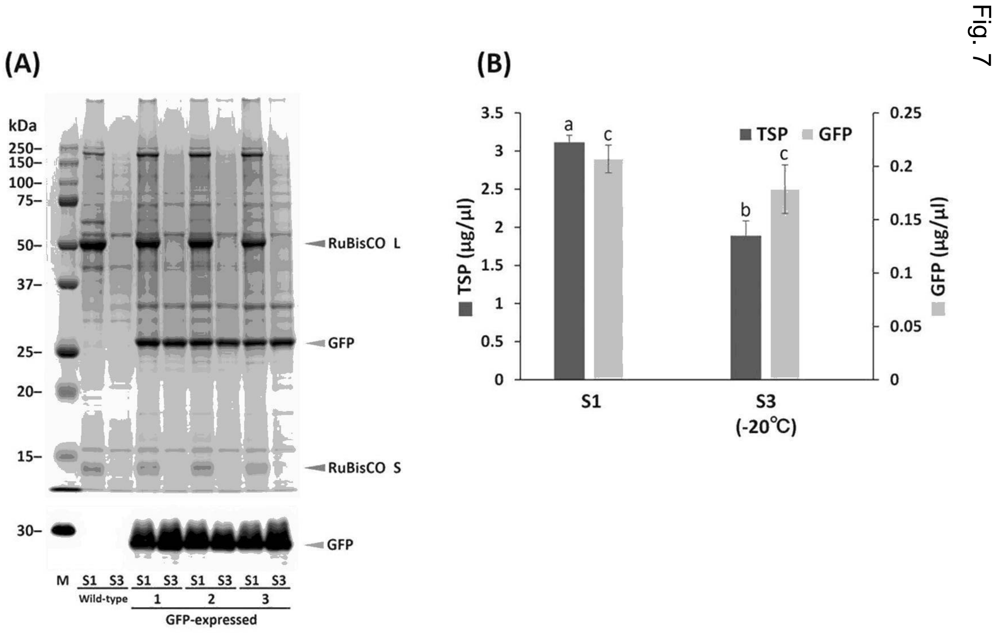

Fig. 7 is a diagram showing the effect of freeze-thaw treatment using -20°C as the freezing temperature. Crude extracts from wild-type and three independent GFP-expressing leaves were frozen at -20°C for 1 day, and then thawed. (A) Proteins were separated by SDS-PAGE and detected by Coomassie Blue staining (top) or Western blot (bottom). M is the molecular weight marker, RuBisCO L is the RuBisCO large subunit, RuBisCO S is the RuBisCO small subunit, S1 is the unfrozen supernatant, and S3 is the supernatant of the crude extract frozen at -20°C for 1 day. (B) Concentration of TSP and GFP in the corresponding supernatants.

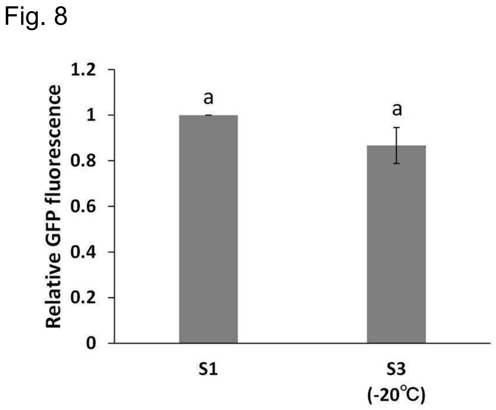

Fig. 8 is a diagram showing the effect of freeze-thaw treatment using -20°C as the freezing temperature on the function of GFP. It shows the relative GFP fluorescence intensity of S1 and S3 from GFP-expressing plants. S1 is the unfrozen supernatant, and S3 is the supernatant of the crude extract frozen at -20°C for 1 day.

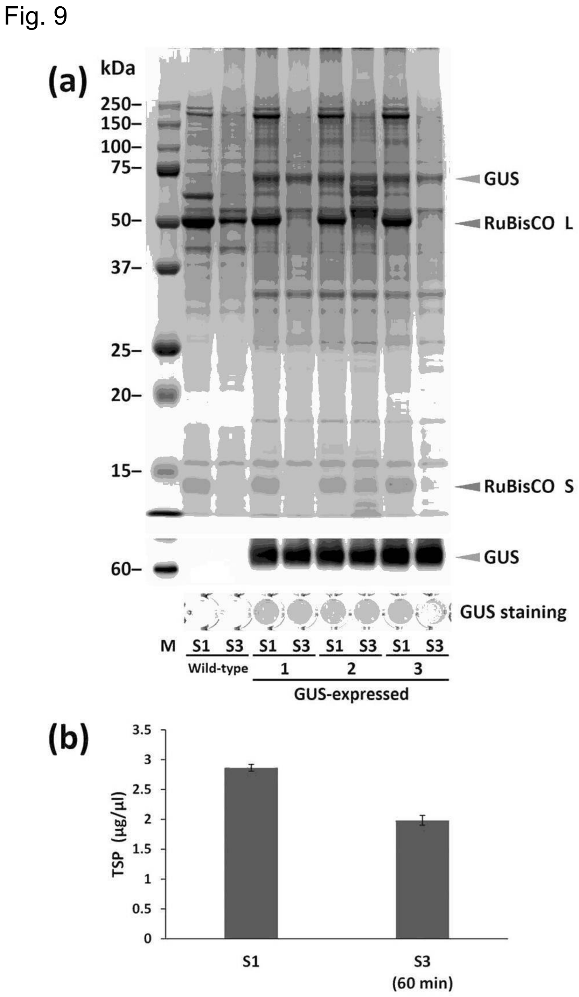

Fig. 9 is a diagram showing the effect of freeze-thaw treatment on GUS (β-glucuronidase) protein. Crude extracts from wild-type and three independent GUS-expressing leaves were frozen at -30°C for 60 minutes, and then thawed. (A) Proteins were separated by SDS-PAGE and detected by Coomassie Blue staining (top) or Western blot (middle). GUS staining results are also shown (bottom). (B) TSP concentration in the corresponding supernatants. M is the molecular weight marker, RuBisCO L is the RuBisCO large subunit, RuBisCO S is the RuBisCO small subunit, S1 is the unfrozen supernatant, and S3 is the supernatant of the crude extract frozen at -30°C for 60 minutes.

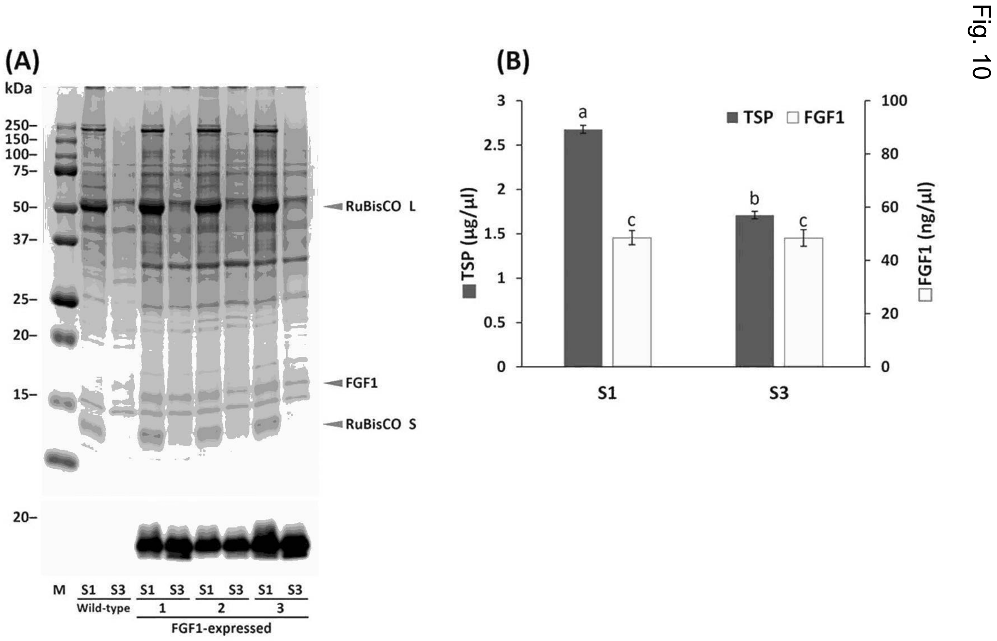

Fig. 10 is a diagram showing the effect of freeze-thaw treatment on human FGF1 protein. Crude extracts from wild-type and three independent FGF1-expressing leaves were frozen at -30°C for 1 day, and then thawed. (A) Proteins were separated by SDS-PAGE and detected by Coomassie Blue staining (top) or Western blot (bottom). M is the molecular weight marker, RuBisCO L is the RuBisCO large subunit, RuBisCO S is the RuBisCO small subunit, S1 is the unfrozen supernatant, and S3 is the supernatant of the crude extract frozen at -30°C for 1 day. (B) Concentration of TSP and FGF1 in the corresponding supernatants.

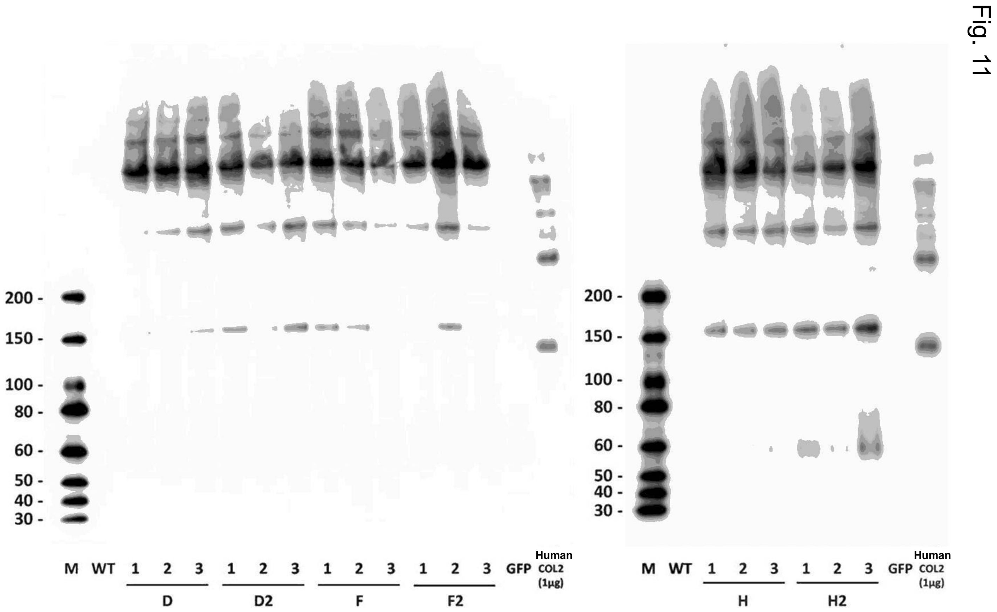

Fig. 11 is a diagram showing the extraction and confirmation of expression of human Type II Collagen protein expressed by a transient expression system in *N*. *benthamiana*. Crude extracts obtained from leaves infected with *Agrobacterium* (strains D, D2, F, F2, H, H2; n=3) encoding various designed human Type II Collagens were separated by non-reducing SDS-PAGE, and human Type II Collagen was detected by Western blot. Negative controls are "WT" (extract from uninfected plants) and "GFP" (extract from leaves infected with *Agrobacterium* encoding GFP). The positive control was human Type II Collagen protein derived from human tissue ("Human COL2 (1µg)"). M indicates the molecular weight marker.

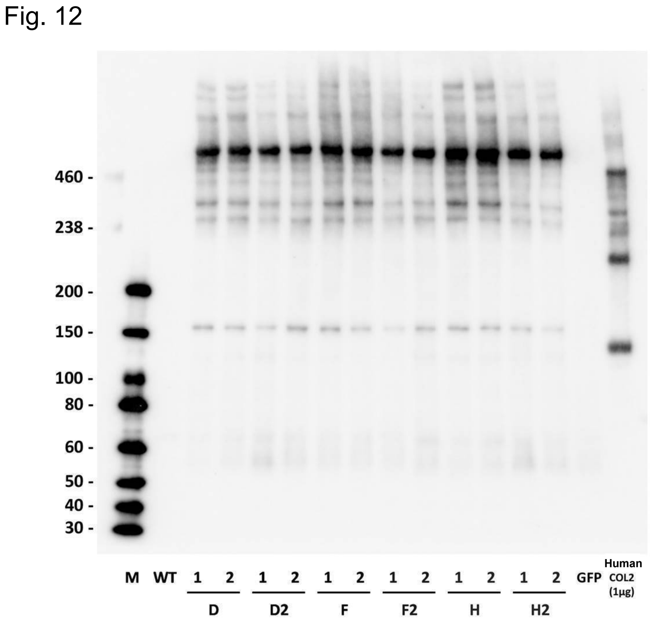

Fig. 12 shows the results demonstrating the application of freeze-thaw treatment to human Type II Collagen protein. Samples were analyzed by analyzing the supernatant obtained by freezing the crude juice at -30°C for 2 weeks, followed by thawing and centrifugation. The figure shows the analysis results of detecting human Type II Collagen by Western blot after separation by non-reducing SDS-PAGE. (Samples prepared under the same conditions as in Fig. 11 were subjected to freeze-thaw treatment; n=2).

### Description of Embodiments

The present invention provides a method for removing a plant-derived soluble protein from a plant-derived extract (hereinafter, the removal method of the present invention).

The removal method of the present invention includes a step of insolubilizing a plant-derived soluble protein by freezing and thawing a plant-derived extract (hereinafter, step (1)).

In step (1), the plant from which the plant-derived extract is extracted is not particularly limited, but model plants for which cultivation techniques, breeding techniques, or gene introduction techniques have been established are envisaged. Examples of such plants include plants belonging to the genus *Arabidopsis* (e.g., *Arabidopsis thaliana*), the genus *Oryza* (e.g., *Oryza sativa*), the genus *Triticum* (e.g., *Triticum aestivum*), the genus *Brachypodium* (e.g., *Brachypodium distachyon*), and the genus *Nicotiana* (e.g., *N. benthamiana*). Furthermore, the above-mentioned plant may be a wild type, or it may be a genetically modified type capable of expressing an exogenous soluble protein that is not insolubilized by the freezing and thawing of step (1).

The aforementioned exogenous soluble protein that is not insolubilized by freezing and thawing is not limited as long as it is a soluble protein and is not insolubilized by the freezing and thawing of step (1) described later. Here, a soluble protein refers to a protein that dissolves well in water, in which the affinity with water molecules is increased by having a large number of positively charged basic amino acid residues, negatively charged acidic amino acid residues, or polar amino acid residues on the surface of the soluble protein at around neutral pH, resulting in the binding of water molecules to the dissociable groups of basic or acidic amino acid residues (ionic hydration) or the formation of hydrogen bonds between polar groups (such as OH groups) and water molecules. Basic amino acids include Lys, Arg, and His. Acidic amino acids include Glu and Asp. Polar amino acids include serine and threonine. Furthermore, a protein that is not insolubilized by freezing and thawing refers to a protein whose high-order structure does not change and which does not become insoluble due to aggregation upon freezing and thawing. Examples of such exogenous soluble proteins include antibodies, cytokines (IFN-α, IFN-β, FGF, etc.), various enzymes, viral, bacterial, or cancer antigen proteins, extracellular matrix proteins such as collagen, and high-value-added proteins such as albumin. By using, for example, a part of a viral protein as an antigen, it becomes possible to mass-produce antigens useful in the development of vaccines or antibodies against viruses. Alternatively, it is also possible to mass-produce cancer antigens useful in the development of anti-cancer drugs.

The expression of the exogenous soluble protein may be transient expression or stable expression. Means for expressing the exogenous soluble protein in a plant include methods of introducing an expression vector containing DNA encoding the exogenous soluble protein into plant cells, and methods of introduction using a viral vector having a nucleic acid sequence encoding the exogenous soluble protein. Any known method may be followed as long as it allows the exogenous soluble protein to be expressed within plant cells by culturing the plant cells.

The introduction of an expression vector containing DNA encoding the exogenous soluble protein into plant cells can be carried out by methods known per se, and for example, it can be introduced in the form of an expression vector containing the DNA encoding the exogenous soluble protein. The introduction of the expression vector can be carried out according to known methods (e.g., *Agrobacterium* method, PEG method, electroporation method, particle gun method, whisker direct introduction method, etc.) on appropriate tissues (e.g., callus, root, leaf, seed, growing point, etc.) depending on the type of plant. Among these, the *Agrobacterium* method is particularly preferable because it is easy to scale up. Furthermore, the introduction of the expression vector containing DNA encoding the exogenous soluble protein into plant cells is also possible by using a viral vector having a nucleic acid sequence encoding the exogenous soluble protein. The introduction of the viral vector can be carried out on the above-mentioned tissues according to the viral vector method, depending on the type of plant.

When stably expressing an exogenous soluble protein in a plant, for example, a part of a tobacco tissue (e.g., tobacco leaf) or hypocotyl is excised and infected with *Agrobacterium.* By culturing the tissue or hypocotyl into which the expression vector has been introduced, genetically modified plant cells into whose genome the DNA encoding the exogenous soluble protein has been introduced are selected. Subsequently, by inducing callus from the cells and inducing roots from the callus, a genetically modified plant that stably expresses the exogenous soluble protein can be obtained. Alternatively, callus is infected with *Agrobacterium.* As such *Agrobacterium,* typically, one is used in which the DNA encoding the exogenous soluble protein is introduced into the T-DNA fragment of an *Agrobacterium* expression vector. When using the PEG method or electroporation method, protoplasts are prepared from appropriate cells or tissues according to a conventional method, and the expression vector is introduced into them. In the case of the particle gun method, the expression vector, adsorbed onto gold microparticles, can be introduced into callus, immature embryos, or growing points present in apical or axillary buds using a particle gun. With the particle gun method and the *Agrobacterium* method, the genetically modified plant often becomes a chimera, so it is necessary to use sample cells, such as those in which the DNA is introduced at high frequency into germ line cells, for gene introduction. Examples include embryos, hypocotyl sections, embryogenic callus, and isolated growing points.

The culture of plant cells or tissues into which the expression vector has been introduced can be carried out according to known methods, depending on their type. As the medium used for culture, a solid medium (e.g., agar medium, agarose medium, gellan gum medium, etc.) is preferable. Furthermore, the medium preferably contains carbon sources, nitrogen sources, inorganic substances, etc., necessary for the growth of the genetically modified cells. For example, N6 medium, MS medium, MSR medium, LS medium, B5 medium, etc., are used as basal media. Plant growth substances (e.g., auxins, cytokinins, etc.) may be added to the medium as appropriate. The pH of the medium is preferably about 5 to about 8. The culture temperature can be appropriately selected within the range of, usually, about 20°C to about 35°C, depending on the type of plant cell.

The selection of genetically modified plant cells can be carried out by a person skilled in the art by appropriately selecting known techniques according to the type of marker gene used. For example, when using drug resistance genes (e.g., kanamycin resistance gene (nptII), hygromycin resistance gene (hpt), etc.), it is possible to select genetically modified plant cells by culturing them in the presence of the corresponding drug.

When transiently expressing an exogenous soluble protein in a plant, for example, a method of immersing the entire tobacco plant in a suspension of *Agrobacterium,* sealing, and applying vacuum (e.g., 0.09 MPa) (vacuum infiltration method), or a method of infiltrating the *Agrobacterium* suspension into the intercellular spaces of the leaf using a needleless syringe (syringe infiltration method) can be mentioned. After the treatment, by culturing the tobacco tissue or the entire tobacco plant for several days, it is possible to obtain a plant that transiently expresses the exogenous soluble protein in the Agrobacterium-infiltrated tobacco tissue.

In step (1), the acquisition of the plant-derived extract may be carried out according to known means. In the present invention, a plant-derived extract refers to "a suspension obtained by adding water or a buffer solution to crushed plant tissue". For example, a plant extract can be prepared by crushing all or part of a plant (root, leaf, stem, or flower) and suspending it in water or a buffer solution (e.g., Tris-HCl buffer, phosphate buffer, HEPES buffer, etc.). Furthermore, the extract may also contain surfactants (e.g., Triton X-100(trademark), Tween 20(trademark), etc.) or protease inhibitors.

The plant-derived extract obtained as described above contains soluble substances such as endogenous and exogenous soluble proteins, as well as insoluble substances such as crushed fragments. Therefore, the removal method of the present invention may further include a step of removing insoluble substances from the plant-derived extract before step (1). The step of removing insoluble substances from the plant-derived extract is not particularly limited as long as insoluble substances can be removed, but preferable examples include a step of removing insoluble substances from the plant-derived extract by centrifugation. The centrifugation conditions are not particularly limited as long as they are conditions under which insoluble substances precipitate, but examples include about 10,000 to about 30,000 ×g, about 2 to about 20 minutes, about 0 to about 4°C, and the like. Centrifugation is a simple and rapid method, offering good recovery of soluble substances, using a general-purpose machine (centrifuge), having a short processing time, and allowing precipitates to be determined simply based on molecular weight. Furthermore, another step for removing insoluble substances from the plant-derived extract includes a step of removing insoluble substances by filtering the plant-derived extract. The filtration membrane used for filtration is not particularly limited as long as it can filter insoluble substances, but examples include gauze, filter paper, and the like. The pore size of the filtration membrane is not particularly limited as long as it is a size that can filter insoluble substances, but examples include 10 µm to 700 µm. By removing the obtained precipitate, it is possible to remove insoluble substances from the plant-derived extract in advance.

In step (1), the plant-derived extract is subjected to freezing treatment. The freezing conditions are not particularly limited as long as the extract is frozen. For example, the range of -30°C to -20°C can be mentioned, but the conditions are not limited thereto. Furthermore, the freezing period is not limited as long as it is a time for the plant-derived extract to freeze. For example, the range of 10 minutes to 14 days can be mentioned. Alternatively, the extract may be instantaneously frozen using liquid nitrogen.

In one aspect, if the exogenous soluble protein loses some or all of its biological activity due to freezing, shortening the freezing period may avoid the loss of biological activity. When using an exogenous soluble protein with such characteristics, the freezing period for the plant-derived extract can be, but is not limited to, typically 10 minutes to 12 hours, preferably 10 minutes to 11 hours, 10 minutes to 10 hours, 10 minutes to 9 hours, 10 minutes to 8 hours, 10 minutes to 7 hours, 10 minutes to 6 hours, 10 minutes to 5 hours, 10 minutes to 4 hours, 10 minutes to 3 hours, 10 minutes to 2 hours, or 10 minutes to 1 hour.

In step (1), the frozen plant-derived extract is thawed. The thawing conditions are not particularly limited as long as the frozen extract is thawed. The thawing temperature is, for example, 4°C.

As described above, by freezing and thawing the plant-derived extract, it is possible to insolubilize the plant-derived soluble proteins contained in the plant-derived extract. Examples of plant-derived soluble proteins include RuBisCO. RuBisCO is the only enzyme involved in the carbon fixation reaction in the Calvin cycle of plant photosynthesis, and it catalyzes the reaction that fixes carbon dioxide to ribulose-1,5-bisphosphate to produce two molecules of 3-phosphoglycerate. RuBisCO is the most abundant protein in plants and is the protein most removed during the process of purifying proteins other than RuBisCO from plants. RuBisCO normally exists as a hetero-16-mer consisting of eight large subunits and eight small subunits, and is present as a soluble protein in the plant-derived extract. The freeze-and-thaw treatment of step (1) is thought to change the high-order structure of the RuBisCO large subunits, small subunits, or the hetero-16-mer composed of them, causing insolubilization. In addition, when the plant expresses an exogenous soluble protein that is not insolubilized by freezing and thawing, the exogenous soluble protein contained in the plant-derived extract is not insolubilizing by the above-mentioned freezing and thawing, so the plant-derived soluble protein and the exogenous soluble protein can be separated.

The separation method of the present invention includes a step of removing the insolubilized protein from the freeze-and-thawed plant-derived extract (hereinafter, step (2)).

The step of removing the insolubilized protein from the freeze-and-thawed plant-derived extract is not particularly limited as long as the insolubilized protein can be removed, and preferable examples include a step of removing the insolubilized protein by centrifuging the freeze-and-thawed plant-derived extract. The centrifugation conditions are not particularly limited as long as they are conditions under which the insolubilized protein precipitates. For example, about 10,000 to about 30,000 ×g, about 2 to about 20 minutes, about 0 to about 4°C and the like can be mentioned. By removing the obtained precipitate, it is possible to separate the insolubilized protein from the plant-derived extract. Furthermore, another step for removing the insolubilized protein from the freeze-and-thawed plant-derived extract includes a step of removing the insolubilized protein by filtering the freeze-and-thawed plant-derived extract. The filtration membrane used for filtration is not particularly limited as long as it can filter the insolubilized protein, and examples include gauze, filter paper, and the like. The pore size of the filtration membrane is not particularly limited as long as it is a size that can filter the insolubilized protein, and examples include 10 µm to 700 µm. When the plant expresses an exogenous soluble protein that is not insolubilized by the freezing and thawing of step (1), it is possible to separate the insolubilized protein from the plant-derived extract and increase the proportional presence of the exogenous soluble protein among the soluble proteins contained in the plant-derived extract.

Hereinafter, the present invention will be described by way of examples. However, the present invention is not limited to these examples.

### Examples

### Example 1

Verification of the separation effect of plant-derived soluble protein and exogenous soluble protein by freeze-thaw treatment

*Agrobacterium* into which a plant expression vector expressing GFP (GFP pBE2113) was introduced was shake-cultured overnight at 28°C, 220 rpm in 3 ml of YEP medium (containing kanamycin (50 µg/mL), streptomycin (300 µg/mL), rifampicin (100 µg/mL)). The *Agrobacterium* was collected by centrifuging the culture solution at 5,000 ×g for 10 minutes at room temperature (23°C). The collected *Agrobacterium* was resuspended in about 5-10 mL of suspension buffer (10 mM MES-KOH (pH 5.7), 10 mM MgCl2, 150 µM acetosyringone (added immediately before use)) and further diluted with the same suspension buffer to an OD600=0.5. The suspension was allowed to stand for about 2 hours and infiltrated from the underside of *N. benthamiana* leaves using a needleless 1 mL syringe (syringe infiltration method). After roughly removing the suspension from the leaves, the plants were allowed to stand for about 2 to 3 hours and then grown for 4 days in a chamber (23°C, 16-hour light period, 8-hour dark period).

Wild-type *N. benthamiana* leaves (100 mg) and GFP gene-introduced *N. benthamiana* leaves (100 mg) as described above were each crushed by a multi-bead shocker (1,700 rpm, 30 sec). The crushed material was suspended in 500 µl of pH 7.2 phosphate buffer (0.1% (v/v) Tween 20, 1% (v/v) protease inhibitor cocktail (Sigma-Aldrich #P9599)) to obtain crude extracts of each plant body. The obtained crude extracts were centrifuged (23,000 ×g, 10 min, 2°C) to obtain supernatant (S1) and precipitate (P1). Furthermore, separately prepared supernatant (S1) was left standing at - 30°C (1 day), then thawed in a refrigerator (4°C) and centrifuged (23,000 ×g, 10 min, 2°C) to obtain supernatant (S2) and precipitate (P2). In addition, separately prepared crude extract was left standing at -30°C (1 day), then thawed in a refrigerator (4°C) and centrifuged (23,000 ×g, 10 min, 2°C) to obtain supernatant (S3) and precipitate (P3). Specifically, the flowchart in Fig. 1 was followed.

For both the wild-type and GFP gene-introduced plants, the crude extract was centrifuged to remove the insoluble fraction P1, and the resulting S1 was subjected to freeze-thaw treatment and centrifugation. As a result, a new precipitate P2 was formed (Fig. 2A). This suggested that a portion of the soluble substances remaining in the supernatant before the freeze-thaw treatment was insolubilized by the freeze-thaw treatment.

Furthermore, when the colors of the supernatants were compared under blue LED light, S1 to S3 from the wild-type showed no fluorescence, but GFP fluorescence was observed in all of S1 to S3 obtained from the GFP gene-introduced plants, and the fluorescence intensity was similar between S1 (before freeze-thaw) and S2 (after freeze-thaw) (Fig. 2B). This indicated that the GFP, which was not precipitated by centrifugation and remained in the supernatant before the freeze-thaw treatment, remained in the supernatant without being insolubilized even by the freeze-thaw treatment, and as a result, the substances insolubilized by the freeze-thaw treatment and GFP could be separated.

Proteins contained in S1 to S3 obtained from wild-type and GFP gene-introduced plants were separated by SDS-PAGE, and the polyacrylamide gel was stained with Coomassie Blue. As a result, in S1, bands for the plant-derived soluble protein RuBisCO (L/S) (approx. 10-something kDa (RuBisCO S) and 50 kDa (RuBisCO L)) were detected, but in S2 and S3, these bands were not detected (Fig. 2C). Furthermore, when the amount of GFP contained in S1 to S3 (including the GFP-containing ones) was analyzed by Western blot, no GFP bands could be detected from S1 to S3 of the wild-type, but GFP bands were detected in all of S1 to S3 from the GFP gene-introduced type, and no difference in band intensity was observed among S1 to S3. This also indicated that RuBisCO (L/S), which remained in the supernatant before freezing and thawing, was insolubilized by the freeze-thaw treatment, while GFP remained in the supernatant without being insolubilized even by the freeze-thaw treatment, and as a result, RuBisCO (L/S) and GFP could be separated by the freeze-thaw treatment.

Furthermore, from the results of the Western blot analysis, the amounts of GFP contained in S1 to S3 of the GFP gene-introduced type were compared relatively. As a result, when the amount of GFP in S1 was set to 1, the amount of GFP remaining in S2 was over 0.9 (Fig. 3A). On the other hand, from the Coomassie Blue staining results, when the amounts of RuBisCO contained in S1 to S3 of the GFP gene-introduced type were compared relatively, and the amount of RuBisCO in S1 was set to 1, the amount in S2 was less than 0.1 (Fig. 3B). Furthermore, from the Coomassie Blue staining results, the total protein amounts contained in S1 to S3 of the GFP gene-introduced type were compared relatively. As a result, when the total protein amount in S1 was set to 1, the amount in S2 was less than 0.3 (Fig. 3C). From these results, it was found that by passing through the freeze-thaw treatment, over 70% of the total protein from the crude extract of *N. benthamiana* leaves was removed, and for RuBisCO alone, over 90% was removed. Furthermore, it was also found that GFP, a transiently expressed soluble protein, was hardly affected by the freeze-thaw treatment and remained in the supernatant. Therefore, the freeze-thaw treatment can conveniently and rapidly insolubilize plant-derived soluble proteins from a plant's crude extract, allowing them to be easily precipitated and removed by centrifugation. Furthermore, when expressing an exogenous soluble protein, the freeze-thaw treatment can easily and dramatically improve the purification rate of the target soluble protein. Moreover, similar results were obtained even without performing the operation of centrifuging the plant crude extract and removing the insoluble fraction in advance before implementing the freeze-thaw treatment.

### Example 2

Verification of the effect of freezing period on the separation of plant-derived soluble protein and exogenous soluble protein 1

Similarly to Example 1, wild-type *N. benthamiana* leaves (100 mg) and leaves obtained from GFP-introduced *N*. *benthamiana* (100 mg) were each crushed by a multi-bead shocker (1,700 rpm, 30 sec), and the crushed material was suspended in 500 µl of phosphate buffer (0.1% (v/v) Tween 20, with protease inhibitor) to obtain crude extracts of each plant body. The obtained crude extracts were immediately centrifuged (23,000 ×g, 10 min, 2°C) to obtain supernatant S1 and precipitate P1. Furthermore, supernatant S1 was left standing at -30°C (1 or 7 days), thawed in a refrigerator (4°C), and centrifuged (23,000 ×g, 10 min, 2°C) to obtain supernatant S2 (frozen 1 day), supernatant S2 (frozen 7 days), and precipitate P2 (frozen 1 day), precipitate P2 (frozen 7 days).

When the colors of the precipitates were compared under visible light, for both wild-type and GFP gene-introduced types, P1 obtained by centrifuging the crude extract was green, but P2 (frozen 1 day) and P2 (frozen 7 days) obtained by centrifuging S1 were both white, and no difference was observed (Fig. 4A). This suggested that the freezing period does not affect the precipitate.

Furthermore, when the colors of the supernatants were compared under blue LED light, S1, S2 (frozen 1 day), and S2 (frozen 7 days) from the wild-type showed no GFP fluorescence, but fluorescence was observed in all of S1, S2 (frozen 1 day), and S2 (frozen 7 days) obtained from the GFP gene-introduced plants, and their fluorescence intensities were similar (Fig. 4B). This suggested that the freezing period does not affect the state of GFP.

Proteins contained in S1, S2 (frozen 1 day), and S2 (frozen 7 days) of the wild-type and GFP gene-introduced types were separated by SDS-PAGE and subjected to Coomassie Blue staining and Western blot analysis. As a result, in S1, bands for RuBisCO (L/S) were detected, but in S2 (frozen 1 day) and S2 (frozen 7 days), these bands were not detected (Fig. 4C). Furthermore, no GFP bands could be detected from S1, S2 (frozen 1 day), and S2 (frozen 7 days) of the wild-type, but GFP bands were detected in all of S1, S2 (frozen 1 day), and S2 (frozen 7 days) obtained from the GFP gene-introduced plants, and no difference in band intensity was observed among S1, S2 (frozen 1 day), and S2 (frozen 7 days). In fact, from the results of the Western blot analysis, when the amounts of GFP contained in S1, S2 (frozen 1 day), and S2 (frozen 7 days) of the GFP gene-introduced type were compared relatively, no difference was observed in the respective GFP amounts. These results also suggested that the freezing period does not affect the precipitate or the state of GFP. It was shown that the freeze treatment can insolubilize proteins, including RuBisCO, from the plant crude extract, allowing them to be easily removed by centrifugation or the like. Furthermore, it was shown that when expressing an exogenous soluble protein, the purification rate of the target soluble protein can be easily and dramatically improved by the freeze treatment.

### Example 3

Verification of the effect of freezing period on the separation of plant-derived soluble protein and exogenous soluble protein 2

An experiment as in Example 2 was conducted, except that the freezing period of the crude extract was set to 10 minutes or 60 minutes, to evaluate the effect of short-time freezing. The results are shown in Fig. 5.

As shown in Fig. 5, 10 minutes of freezing showed a weak, but present, RuBisCO removal effect. Furthermore, 60 minutes of freezing showed a sufficient RuBisCO removal effect. In addition, no effect on GFP was observed in the freeze-thaw treatment under these conditions (Fig. 5 (B) and Fig. 6).

### Example 4

Verification of the effect of freezing temperature on the separation of plant-derived soluble protein and exogenous soluble protein

An experiment as in Example 3 was conducted, except that the freezing period of the crude extract was 1 day and the freezing temperature was -20°C, to evaluate the effect when -20°C was adopted as the freezing temperature. The results are shown in Fig. 7 and Fig. 8.

As shown in Fig. 7 and Fig. 8, even when the freezing temperature was -20°C, results similar to those when the freezing temperature was -30°C were obtained. Specifically, the amounts of total soluble proteins (TSP) and RuBisCO in the supernatant decreased, but no effect on GFP was observed.

### Example 5

Investigation of the effect of freeze-thaw treatment on proteins other than GFP 1 (GUS)

The effect of freeze-thaw treatment on transiently expressed GUS (β-glucuronidase) was evaluated. A crude extract of GUS-expressing leaves was frozen at -30°C for 60 minutes and thawed at 4°C. As a result of Western blot analysis, GUS expression was observed in all S1 and S3 samples, and GUS activity was detected in the supernatant (Fig. 9(a)). Even with 60 minutes of freezing, TSP decreased sufficiently (Fig. 9(b)). This suggests that RuBisCO can be removed with a shorter freezing time without inactivating freeze-sensitive proteins.

### Example 6

Investigation of the effect of freeze-thaw treatment on proteins other than GFP 2 (Human FGF1)

The effect of freeze-thaw treatment on transiently expressed human FGF1 was evaluated. A crude extract of FGF1-expressing leaves was frozen at -30°C for 1 day and thawed at 4°C. FGF1 was detected by SDS-PAGE and Western blot analysis (Fig. 10(A)). ELISA analysis showed almost no change in the amount of FGF1 after the freeze-thaw treatment (Fig. 10(B)).

### Example 7

Investigation of the effect of freeze-thaw treatment on proteins other than GFP 3 (Human Collagen II)

The effect of freeze-thaw treatment on transiently expressed human Collagen II was evaluated. Human Collagen II in a crude extract of human Collagen II-expressing leaves was detected by Western blot analysis (Fig. 11). As a result, bands specific to human Collagen II were detected.

A crude extract of human Collagen II-expressing leaves was frozen at -30°C for 2 weeks, then thawed at 4°C, and human Collagen II was detected by Western blot analysis (Fig. 12). As a result, bands specific to human Collagen II were detected after freezing and thawing, similar to the sample before freezing.

### Example 8

### Proteome Analysis

To investigate the effect of the freeze-thaw treatment, a proteome analysis was performed on the supernatant of wild-type plants before and after the freeze-thaw treatment. As a result, 695 proteins were identified in the test supernatant. The average abundance ratio of three proteins was 1 or more, and the abundance ratio of 692 proteins was less than 1. Table 1 shows the top 20 proteins whose amounts decreased after the freeze-thaw treatment. As shown in Table 1, the amounts of many endogenous proteins in the supernatant after the freeze-thaw treatment were reduced compared to the unfrozen supernatant. In particular, ribosomal proteins were effectively removed from the supernatant after the freeze-thaw treatment. In the table, Abundance Ratio means (abundance after freeze-thaw treatment) / (abundance before freeze-thaw treatment).

**[Table 1-1]**

| Accession | Gene symbol | Description | Coverage [%] | Average abundance ratio | Coefficient of variation [%] |
|---|---|---|---|---|---|
| A0A1S4ALT8 | LOC107799053 | 40S ribosomal protein S26 | 21 | 0.0995 | 19.32333419 |
| | | OS=Nicotiana | | | |
| | | tabacum OX=4097 | | | |
| | | GN=LOC107799053 PE=3 SV=1 | | | |
| A0A1S3Y5V8 | LOC107772468 | 60S acidic ribosomal protein P0 | 11 | 0.1155 | 28.33431718 |
| | | OS=Nicotiana | | | |
| | | tabacum OX=4097 | | | |
| | | GN=LOC107772468 PE=3 SV=1 | | | |
| A0A1S4DQP2 | LOC107832413 | 50S ribosomal protein L22. | 29 | 0.12125 | 20.99856009 |
| | | chloroplastic | | | |
| | | OS=Nicotiana tabacum OX=4097 | | | |
| | | GN=LOC107832413 PE=3 SV=1 | | | |
| A0A1S4C2S1 | LOC107814537 | 40S ribosomal protein S2-3-like | 48 | 0.12675 | 20.0253099 |
| | | OS=Nicotiana | | | |
| | | tabacum OX=4097 | | | |
| | | GN=LOC107814537 PE=3 SV=1 | | | |
| A0A1U7Y628 | LOC104241039 | Catalase OS=Nicotiana sylvestris | 32 | 0.1345 | 28.708899 |
| | | OX=4096 GN=LOC104241039 PE=3 | | | |
| | | SV=1 | | | |
| A0A1S4BWC8 | LOC107812523 | 26S protease regulatory subunit 8 | 16 | 0.13825 | 21.65456402 |
| | | homolog A- like OS=Nicotiana | | | |
| | | tabacum OX=4097 | | | |
| | | GN=LOC107812523 PE=3 SV=1 | | | |
| A0A1S3YUN7 | LOC107779727 | 40S ribosomal protein S16-like | 28 | 0.1385 | 16.48045085 |
| | | OS=Nicotiana tabacum OX=4097 | | | |
| | | GN=LOC107779727 PE=3 SV=1 | | | |
| A0A1S3XQS4 | LOC107767513 | Uncharacterized protein | 16 | 0.14 | 18.32250763 |
| | | LOC107767513 | | | |
| | | OS=Nicotiana tabacum OX=4097 | | | |
| | | GN=LOC107767513 PE=3 SV=1 | | | |
| A0A286RX19 | rps8 | 30S ribosomal protein S8. | 21 | 0.144 | 26.21163667 |
| | | chloroplastic OS=Nicotiana | | | |
| | | attenuata OX=49451 GN=rps8 PE=3 | | | |
| A0A1J6IAA5 | HSC80 0 | Heat shock cognate protein 80 | 37 | 0.146 | 28.01249844 |
| | | OS=Nicotiana attenuata OX=49451 | | | |
| | | GN=HSC80_0 PE=3SV=1 | | | |

**[Table 1-2]**

| Accession | Gene symbol | Description | Coverage [%] | Average abundance ratio | Coefficient of variation [%] |
|---|---|---|---|---|---|
| A0A1S3Z334 | LOC107782473 | Chloroplast stem-loop binding | 52 | 0.149 | 25.97584226 |
| | | protein of 41 kDa b. chloroplastic | | | |
| | | OS=Nicotiana tabacum OX=4097 | | | |
| | | GN=LOC107782473 PE=4 SV=1 | | | |
| A0A1J6JW23 | RPS24A_1 | 40S ribosomal protein S24 | 36 | 0.15125 | 20.791435 |
| | | OS=Nicotiana attenuata OX=49451 | | | |
| | | GN=RPS24A_1 PE=3 SV=1 | | | |
| A0A1J6IV14 | RPS25 0 | 40S ribosomal protein S25 | 13 | 0.1535 | 29.29665186 |
| | | (Fragment) OS=Nicotiana attenuata | | | |
| | | OX=49451GN=RPS25_0 PE=3 SV=1 | | | |
| A0A1J6IHN4 | RPS9C_2 | 40s ribosomal protein s9-2 | 37 | 0.15475 | 27.43322462 |
| | | OS=Nicotiana attenuata OX=49451 | | | |
| | | GN=RPS9C_2 PE=3 SV=1 | | | |
| A0A1S4D0A1 | LOC107824561 | Ribosomal protein L15 OS=Nicotiana | 22 | 0.1625 | 25.21575538 |
| | | tabacum OX=4097 | | | |
| | | GN=LOC107824561 PE=3 SV=1 | | | |
| A0A314KXC5 | RPS2D | 40s ribosomal protein s2-4 | 49 | 0.165 | 7.455612965 |
| | | OS=Nicotiana attenuata OX=49451 | | | |
| | | GN=RPS2D PE=3 SV=1 | | | |
| A0A1S4DFI4 | LOC107829071 | 40S ribosomal protein S15-like | 24 | 0.16525 | 18.92062273 |
| | | OS=Nicotiana tabacum OX=4097 | | | |
| | | GN=LOC107829071 PE=3 SV=1 | | | |
| A0A1S3XI79 | LOC107765428 | 60S ribosomal protein L5-like | 17 | 0.1655 | 11.5543279 |
| | | OS=Nicotiana tabacum OX=4097 | | | |
| | | GN=LOC107765428 PE=3 SV=1 | | | |
| AOA314KK41 | RPL38 0 | 60s ribosomal protein 138 (Fragment) | 14 | 0.16975 | 26.72632708 |
| | | OS=Nicotiana attenuata | | | |
| | | OX=49451GN=RPL38_0 PE=3 SV=1 | | | |
| A0A1S4B0G2 | LOC107803255 | 60S ribosomal protein L6-like | 26 | 0.17075 | 12.6277753 |
| | | isoform X1 OS=Nicotiana tabacum | | | |
| | | OX=4097 GN=LOC107803255 PE=3 | | | |
| | | SV=1 | | | |

As a result of SDS-PAGE analysis, the amounts of the large and small subunits of RuBisCO contained in the supernatant after freeze-thaw treatment were significantly reduced compared to the unfrozen supernatant. In the frozen samples, the average abundance ratios of the large and small subunits of RuBisCO decreased to about 10% and 20%, respectively, of the unfrozen supernatant solution (Table 2).

**[Table 2]**

| Accession | Gene Symbol | Description | Coverage [%] | Average Abundance Ratio | Coefficient of variation [%] |
|---|---|---|---|---|---|
| Q8SAQ3 | | Ribulose bisphosphate carboxylase small subunit (Fragment) OS=Nicotiana benthamiana | 43 | 0.3475 | 21.29301955 |
| V9IN82 | rbcS | Ribulose bisphosphate carboxylase small subunit. chloroplastic OS=Nicotiana attenuata | 33 | 0.155 | 36.61349533 |
| Q84QE5 | LOC107771723 | Ribulose bisphosphate carboxylase small subunit, chloroplastic OS=Nicotiana tabacum | 41 | 0.152 | 41.95422471 |
| P26573 | RBCS | Ribulose bisphosphate carboxylase small subunit, chloroplastic OS=Nicotiana plumbaginifolia | 36 | 0.151 | 34.98043085 |
| A0A1J6IDB3 | RBCL | Ribulose bisphosphate carboxylase large chain OS=Nicotiana attenuata | 14 | 0.1155 | 30.29478346 |
| A0A7T7D7W9 | rbcL | Ribulose bisphosphate carboxylase large chain OS=Nicotiana suaveolens | 67 | 0.09825 | 42.28004196 |

These values were higher than those estimated from the band intensities observed by SDS-PAGE. In contrast, there were only 15 proteins that were hardly affected by the freeze-thaw treatment (their average abundance ratio was 90% or more) (Table 3). Interestingly, most of the identified proteins were chloroplastic types: A0A1U7YAD0 (Putative inactive purple acid phosphatase 29 isoform X2) and A0A1S3XW44 (GDSL esterase/lipase APG-like) (Table 3).

**[Table 3-1]**

| Accession | Gene Symbol | Description | Coverage [%] | Average Abundance Ratio | Coefficient of variation [%] |
|---|---|---|---|---|---|
| A0A1S3Z1X1 | LOC107781865 | Ribose-5-phosphate isomerase OS=Nicotiana tabacum OX=4097 GN=LOC107781865 PE=3 SV=1 | 48 | 1.41275 | 19.90655729 |
| A0A1J6KQ67 | IN37_1 | 2-methyl-6-phytyl-1.4-hydroquinone methyltransferase, chloroplastic OS=Nicotiana attenuate OX=49451 GN=IN37 1 PE=3 SV=1 | 20 | 1.03675 | 28.89640326 |
| A0A1J6KDK3 | OSI_4 | Putative 6-phosphogluconolactonase 4, chloroplastic OS=Nicotiana attenuata OX=49451 GN=OSI 4 PE=3 SV=1 | 14 | 1.0335 | 20.70761872 |
| A0A1J6I4H2 | PPL1 | 23 kDa subunit of oxygen evolving system of photosystem II OS=Nicotiana attenuata OX=49451 GN=PPL1 PE=3 SV=1 | 25 | 0.99275 | 2.974956318 |
| A0A1U7VKK 3 | LOC104214421 | 2-methylene-furan-3-one reductase OS=Nicotiana sylvestris OX=4096 GN=LOC104214421 PE=3 SV=1 | 54 | 0.9805 | 10.67217287 |
| A0A1S4CXK0 | LOC107823581 | Thylakoid lumenal 17.9 kDa protein. chloroplastic-like isoform X1 OS=Nicotiana tabacum OX=4097 GN=LOC107823581 PE=4 SV=1 | 11 | 0.9535 | 14.94472503 |
| A0A1J6IBG4 | CLPT1 1 | Atp-dependent clp protease atp-binding subunit clpt1, chloroplastic OS=Nicotiana attenuata OX=49451 GN=CLPT1_1 PE=4 SV=1 | 27 | 0.9515 | 8.698977171 |
| A0A314L6A4 | PSBP2 | 23 kDa subunit of oxygen evolving system of photosystem II OS=Nicotiana attenuata OX=49451 GN=PSBP2 PE=3 SV=1 | 54 | 0.936 | 14.93766522 |
| I0B7J6 | psbP2 | 23 kDa subunit of oxygen evolving system of photosystem II OS=Nicotiana benthamiana OX=4100 GN=psbP2 PE=2 SV=1 | 58 | 0.93425 | 12.19772318 |
| A0A314KUY7 | EO_1 | 2-methylene-furan-3-one reductase OS=Nicotiana attenuata OX=49451 GN=EO 1 PE=3 SV=1 | 56 | 0.93325 | 10.90162923 |
| A0A1U7YAD0 | LOC104245036 | Putative inactive purple acid phosphatase 29 isoform X2 OS=Nicotiana sylvestris OX=4096 GN=LOC104245036 PE=4 SV=1 | 18 | 0.92725 | 20.95584863 |
| ADA314L6V8 | FKBP18 | Peptidylprolyl isomerase OS=Nicotiana attenuata OX=49451 GN=FKBP18 PE=4 SV=1 | 17 | 0.9245 | 15.46605539 |

**[Table 3-2]**

| Accession | Gene Symbol | Description | Coverage [%] | Average Abundance Ratio | Coefficient of variation [%] |
|---|---|---|---|---|---|
| A0A1S3XW44 | LOC107769403 | GDSL esterase/lipase APG-like OS=Nicotiana tabacum OX=4097 GN=LOC107769403 PE=3 SV=1 | 18 | 0.9215 | 7.025277617 |
| A0A1S4D251 | LOC107825140 | Ribosome-recycling factor. chloroplastic OS=Nicotiana tabacum OX=4097 GN=LOC107825140 PE=3 SV=1 | 34 | 0.915 | 10.73785555 |
| A0A314KLZ1 | LOC107781865 | Lactoylglutathione lyase OS=Nicotiana attenuate OX=49451 GN=A4A49_20638 PE=3 SV=1 | 28 | 0.90725 | 17.12457951 |

### Industrial Applicability

Whereas conventionally, separation and purification of exogenous soluble proteins expressed in plants required multiple reagents and multi-step operations, according to the present invention, by passing through a freeze-thaw treatment, it becomes possible to remove plant-derived soluble proteins, including the dominant protein in plants, RuBisCO, by insolubilizing them. This can increase the proportional presence of the exogenous soluble protein upstream in the purification process. As a result, it becomes possible to shorten the purification step for the exogenous soluble protein, and the present invention can be useful in the bioindustry field.

This application is based on Japanese Patent Application No. 2023-106995 (filed June 29, 2023) and Japanese Patent Application No. 2023-200214 (filed November 27, 2023), the contents of which are all incorporated herein by reference.

## Claims

1. A method for removing a plant-derived soluble protein from a plant-derived extract, the method comprising the following steps:
(1) a step for insolubilizing the plant-derived soluble protein by freezing and thawing the plant-derived extract, and
(2) a step for removing the insolubilized protein from the extract.

2. The method of Claim 1, wherein the plant-derived soluble protein comprises RuBisCO.

3. The method of Claim 1 or 2, wherein the plant is a plant belonging to the genus *Nicotiana.*

4. The method of Claim 3, wherein the plant belonging to the genus *Nicotiana* is *Nicotiana benthamiana*.

5. The method of Claim 1 or 2, wherein the plant expresses an exogenous soluble protein which is not insolubilized by freezing and thawing.

6. The method of Claim 1 or 2, wherein a freezing period is 1 day or more.

7. The method of Claim 1 or 2, wherein a freezing temperature is -20°C or lower.

8. The method of Claim 1 or 2, wherein the removing of the insolubilized protein from the extract is performed by centrifugation or filtration.

9. The method of Claim 1, further comprising a step of removing insoluble substances from the plant-derived extract before the step (1).

10. The method of Claim 9, wherein the removing of insoluble substances from the plant-derived extract is performed by centrifugation or filtration.
